# EUROPEAN PATENT SPECIFICATION

(11) **EP 0 599 036 B1**
(45) Date of publication and mention of the grant of the patent: **18.12.1996**
(21) Application number: 93116307.5
(22) Date of filing: 08.10.1993
(51) Int. Cl.: C07D 207/34, C07D 207/42, C07D 207/36

(54) **Method for the preparation of 2-aryl-5-trifluoromethyl pyrrole compounds**
Verfahren zur Herstellung von 2-aryl-5-trifluoro-methyl Pyrrole-Verbindungen
Procédé de préparation des composés 2-aryl-5-trifluoro pyrrole

(30) Priority: 25.11.1992 US 981626; 11.12.1992 US 989271
(43) Date of publication of application: 01.06.1994
(73) Proprietor: AMERICAN CYANAMID COMPANY, Wayne New Jersey 07470 (US)
(72) Inventor: Kameswaran, Venkataraman, Princeton Junction, New Jersey 08850 (US)
(74) Representative: Wächtershäuser, Günter, Prof. Dr.

(56) References cited:
- EP-A- 0 487 870
- US-A- 5 010 098
- US-A- 5 128 485
- US-A- 5 225 568
- PYRROLES, Part One, "THE SYNTHESIS AND THE PHYSICAL AND CHEMICAL ASPECTS OF THE PYRROLE RING", John Wiley & Sons

## Description

A wide variety of agronomically harmful insects, acarids and nematodes are efficiently and effectively controlled by 2-aryl-5-trifluoromethyl pyrrole compounds.

It is an object of this invention to provide a convenient, direct method for the preparation of arylpyrrole compounds suitable for use in insect, acarid and nematode control. It is another object to this invention to provide a readily utilizable source of important intermediates in the manufacture of further 2-aryl-5-trifluromethyl pyrrole compounds.

The present invention is directed to a process for the preparation of 2-aryl-5-trifluoromethyl pyrrole compounds of formula I 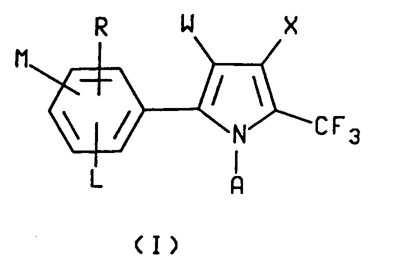 wherein
- A: is hydrogen or C₁-C₆ alkyl optionally substituted with phenyl;
- W: is CN, NO₂, CO₂R₁, or SO₂R1;
- X: is hydrogen or CO₂R₂;
- L: is hydrogen, F, Cl, Br or I;
- M and R: are each independently hydrogen, C₁-C₄ alkyl, C₁-C₄ alkoxy, C₁-C₄ alkylthio, C₁-C₄ alkylsulfinyl, C₁-C₄ alkylsulfonyl, CN, F, Cl, Br, I, NO₂, CF₃, R₃CF₂Z, R₄CO or NR₅R₆ and when M and R are on adjacent positions they may be taken together with the carbon atoms to which they are attached to form a ring in which MR represents the structure
-OCHO-, -OCF₂O- or -CH=CH-CH=CH-;
- R₁: is C₁-C₆ alkyl, C₃-C₆ cycloalkyl or phenyl;
- R₂: is C₁-C₄ alkyl;
- R₃: is hydrogen, F, CHF₂, CHFCl or CF₃;
- R₄: is C₁-C₄ alkyl, C₁-C₄ alkoxy or NR₅R₆;
- R₅: is hydrogen or C₁-C₄ alkyl;
- R₆: is hydrogen, C₁-C₄ alkyl or R₇CO;
- R₇: is hydrogen or C₁-C₄ alkyl;
- Z: is S(O)ₙ or O; and
- n: is an integer of 0, 1 or 2;
which comprises reacting a haloenamine of formula II 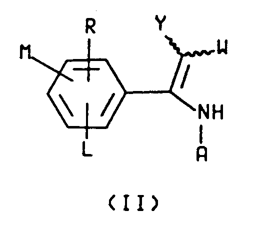 wherein Y is Cl, Br or I, and A, W, L, M and R are as described above with about one molar equivalent of trifluoroacetone or C₁-C₄ alkyl trifluoroacetoacetate in the presence of an acid, and optionally in the presence of a solvent.

The 2-aryl-5-trifluoromethyl pyrrole compounds of formula I are useful as insecticidal, acaricidal and nematicidal agents and as important intermediates in the manufacture of said agents. The utility of the formula I compounds is described in United States Patent No. 5,010,098.

Although there are known methods to synthesize arylpyrrole compounds, commercial-scale manufacturing processes that are economical and environmentally sound are still being sought. The present invention provides a method to obtain useful arylpyrrole compounds and important manufacturing intermediates in a simple single step reaction.

It has now been found that 2-aryl-5-trifluoromethyl pyrrole compounds of formula I may be prepared by reacting an appropriate haloenamine of formula II with about one molar equivalent of trifluoroacetone or C₁-C₄ alkyl trifluoroacetoacetate in the presence of an acid, and optionally in the presence of a solvent, preferably at an elevated temperature. The above reactions are shown below in Flow Diagram I.

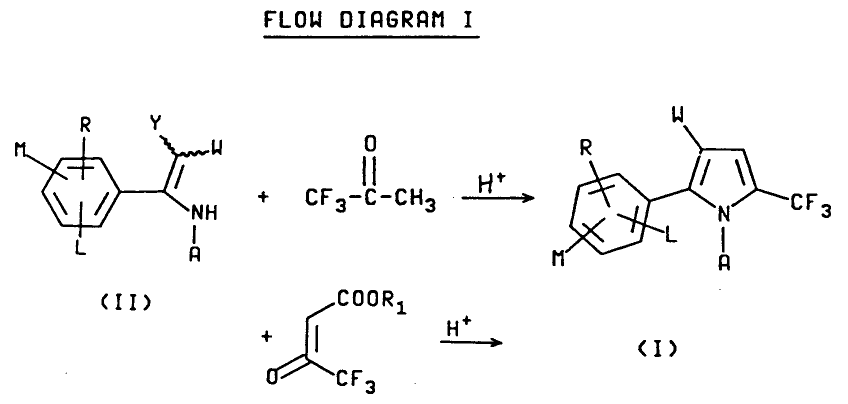

Acids suitable for use in the method of invention are organic acids generally known in the art, in particular carboxylic acids such as acetic acid, propionic acid and the like, preferably acetic acid. A suitable solvent or solvent mixture may optionally be used in conjunction with the organic acid. Such solvents may be selected from organic solvents, such as hydrocarbons, aromatic hydrocarbons, halohydrocarbons, haloaromatic hydrocarbons, and mixtures thereof, which solvent(s) has a boiling point range of about 60° to 250 °C.

The method of invention is most efficient at elevated temperatures such as temperatures in the range of about 60° to about 250°C. Lower reaction temperatures may significantly increase reaction time and excessively high temperatures may lead to unfavorable side-product formation and decomposition. Preferred temperatures are about 70° to about 180°C.

Compounds of formula II may be prepared by reacting the appropriate enamine of formula III with a halogenating agent such as a halogen, an N-halosuccinimide, a hypohalite or the like, as shown in Flow Diagram II.

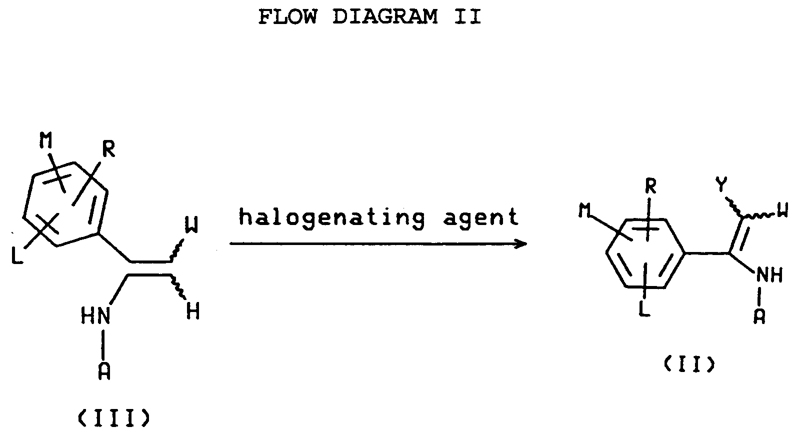

Compounds of formula III and their preparation are described in United States Patent 5,128,485.

The 2-aryl-5-trifluoromethyl pyrrole compounds of formula I are a source of useful intermediates in the manufacture of important arylpyrrole pesticidal agents. For example, in the instance of a formula I compound wherein X is COOR₁, a pesticidal product of formula IV may be obtained directly by a brominative decarboxylation as shown in flow diagram III.

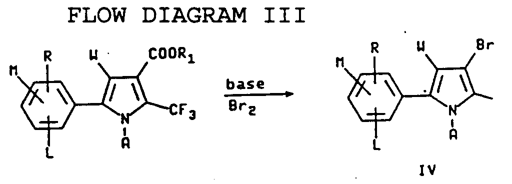

Similarly, a formula I compound wherein X is hydrogen may be readily converted to the desired formula IV product or a 4-halo analogue thereof, by direct halogenation as shown in flow diagram IV.

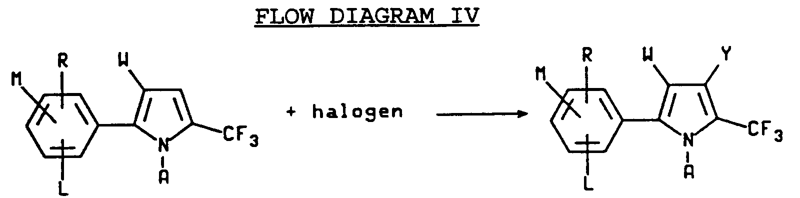

Compounds of fromula IV wherein A is hydrogen may be converted to 1-(alkoxymethyl)pyrrole compounds of formula V via reaction with sodium hydride and a dihalomethane reagent in the presence of an alcohol, R₈OH. The reaction is shown in flow diagram V.

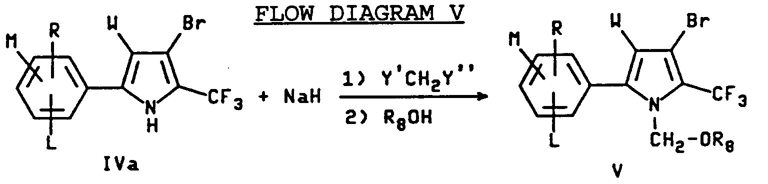

In the above diagram, Y' and Y'' are each independently chlorine, bromine or iodine, R₈ is C₁-C₆ alkyl and W, L, M and R are as described hereinabove for the arylpyrrole compound of Formula I.

Compounds of formula V may also be prepared by the procedure described in United States Patent 5,151,536. Alternatively, the formula V compounds may be obtained by reacting the appropriate pyrrole intermediate of formula IVa with at least 2 molar equivalents of NaH in an aprotic solvent such as tetrahydrofuran to form the corresponding pyrrole anion, treating the anion with at least 1 molar equivalent of a dihalomethane reagent, optionally at an elevated temperature, to form a reaction mixture, and treating the reaction mixture dropwise with an alcohol, R₈OH, so as to maintain a low overall concentration of alcohol in the reaction mixture.

Compounds of formula V, particularly wherein W is CN and R₈ is ethyl, more particularly wherein W is CN, R₈ is ethyl, L and R are hydrogen and M is chlorine, are highly effective insecticidal, acaricidal and nematicidal agents.

As can readily be seen, a wide variety of pesticidal arylpyrroles may be prepared from the intermediate compounds of formula I by varying the substituents, A, L, M, R and W.

In order to facilitate a further understanding of the invention, the following examples are presented to illustrate more specific details thereof. The invention is not to be limited thereby except as defined in the claims. The terms IR and NMR designate infrared and nuclear magnetic resonance, respectively. The term HPLC designates high performance liquid chromatography.

### EXAMPLE 1

### Preparation of 2-(p-Chlorophenyl)-1-methyl-5-(trifluoromethyl) pyrrole-3-carbonitrile

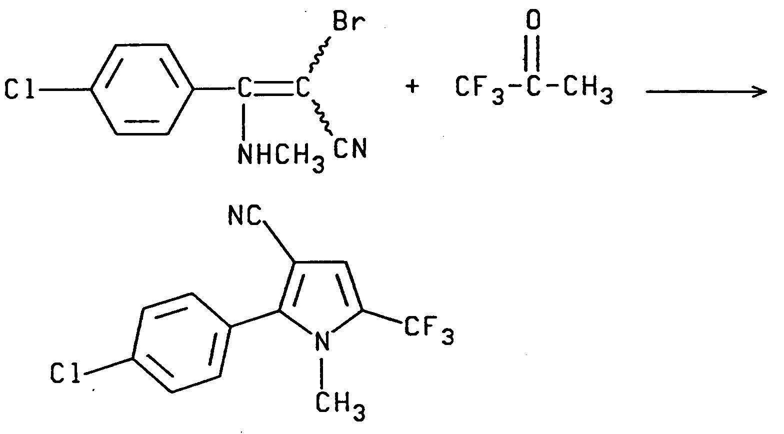

A solution of a-bromo-p-chloro-b-(methylamino)cinnamonitrile, (E)- or (Z)- (5.43 g, 0.02 mol) and trifluoroacetone (3.36 g, 2.7 mL, 0.03 mol) in acetic acid is heated at 80°C for 1-2 hours, heated at 100°C overnight, diluted with water and extracted with ethyl acetate. The combined organic extracts are washed with water, dried over anhydrous Na₂SO₄ and concentrated in vacuo to obtain a gum. The gum is flash chromatographed using silica gel and a 15% ethyl acetate in heptane solution to give the title product as a yellow solid (1.7 g, mp 129°-131°C) which is identified by ¹H and ¹⁹FNMR spectral analyses.

### EXAMPLE 2

### Preparation of 2-(p-Chlorophenyl)-5-(trifluoromethyl) pyrrole-3-carbonitrile

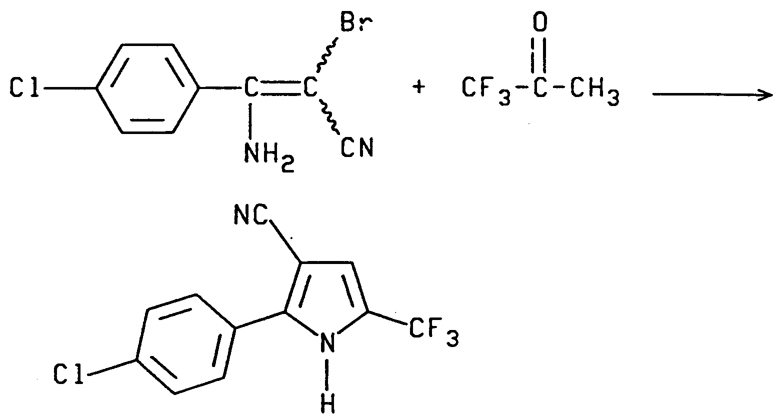

A solution of trifluoroacetone (3.36 g, 2.7 mL,0.03 mol) in acetic acid is added dropwise at 100^{o}C to a solution of β-amino-α-bromo-p-chlorocinnamonitrile, (E)- or (Z)- (5.15 g, 0.02 mol) in acetic acid over 4 1/2 hours. The reaction mixture is heated at 100°C overnight, diluted with water and extracted with ethyl acetate. The combined organic extracts are washed with water, dried over anhydrous Na₂SO₄ and concentrated in vacuo to obtain a gum. The gum is flash chromatographed using silica gel and a 15%- ethyl acetate in heptane solution to give the title product as a yellow solid (1.8 g, mp 129°-131°C) which is identified by ¹H and ¹⁹FNMR spectral analyses.

### EXAMPLE 3

### Preparation of α-Bromo-p-chloro-β-(methylamino)cinnamontrile, (E) - or (Z) -

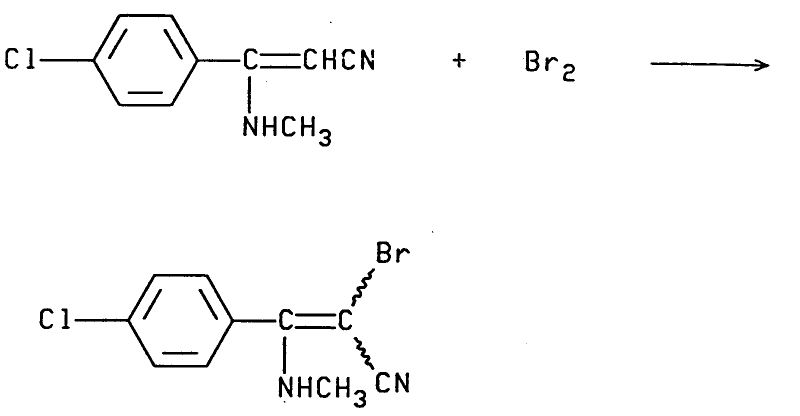

A solution of bromine (10.9 mL, 33.6 g, 0.21 mol) in carbon tetrachloride is added to a mixture of p-chloro-β-(methylamino)cinnamonitrile, (E)- or (Z)-(38.53 g, 0.2 mol) in carbon tetrachloride and tetrahydrofuran at 55°-60°C over 30 minutes. The reaction mixture is cooled to room temperature, concentrated in vacuo to one-half of the original volume and filtered to obtain a solid. The solid is diluted with carbon tetrachloride and the resulting mixture is heated at reflux, cooled and filtered to give the title product as a yellow solid (43.6 g, mp 178°-178.5°C) which is identified by ¹H and ¹³CNMR spectral analyses.

Using essentially the same procedure, but substituting β-amino-p-chlorocinnamonitrile for p-chloro-β-(methylamino)cinnamonitrile, (E)- or (Z)-, β-amino-α-bromo-p-chlorocinnamonitrile, (E)- or (Z)- is obtained as a white solid, mp 190°-191.5°C.

### EXAMPLE 4

### Preparation of α-Bromo-p-chloro-β-(methylamino) cinnamonitrile,E or Z isomer

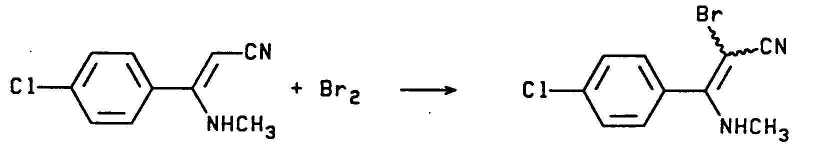

A solution of p-chloro-β-(methylamino)-cinnamonitrile (19.3 g, 0.1 mol) in carbon tetrachloride at 70°C is treated dropwise with a solution of bromine (16.8 g, 0.105 mol) in carbon tetrachloride over a 30-40 minute period (tetrahydrofuran is added as needed during the bromine addition to aid dissolution of the reaction mixture). The reaction mixture is stirred for 30 minutes at 70°C, cooled to room temperature and concentrated to give a yellow crystalline precipitate. The mixture is filtered, the filter cake is washed with carbon tetrachloride and dried to give the title product as a yellow solid, 18.8 g (69% yield), mp 178.0-178.5°C, identified by IR, NMR and mass spectral analyses.

### EXAMPLE 5

### Preparation of 2-(p-chlorophenyl)-1-methyl-5-(trifluoromethyl) pyrrole-3-carbonitrile (I) and 4-carbethoxy-2-(p-chlorophenyl)-1-methyl-5-(trifluoromethyl)pyrrole-3-carbonitrile (II)

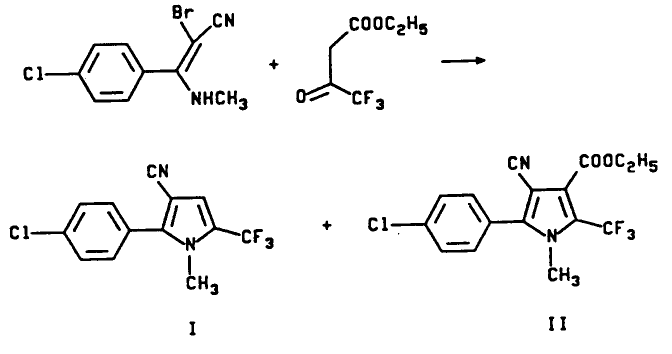

A stirred mixture of 2-bromo-p-chloro-β-(methylamino)cinnamonitrile (10.0 g, 0.0368 mol) and ethyl trifluoroacetoacetate (10.17 g, 0.0552 mol) in acetic acid is heated at 116°-118° for 7 hours, cooled to room temperature, diluted with water and extracted with ethyl acetate. The extracts are combined, washed with water, dried over Na₂SO₄ and concentrated in vacuo to give a gum residue. The residue is flash chromatographed using silica gel and 15:1 ethyl acetate:heptane as eluent to give the title product (I) as a white solid, 2.7 g, mp 127-129°C identified by ¹HNMR and ¹⁹FNMR spectral analyses and the other title product (II) as orange crystals, 1.65 g., mp 129°-131°C, identified by ¹HNMR and ¹⁹FNMR spectral analyses.

### EXAMPLE 6

### Preparation of 4-bromo-2-(p-chlorophenyl)-5-(trifluoromethyl) pyrrole-3-carbonitrile

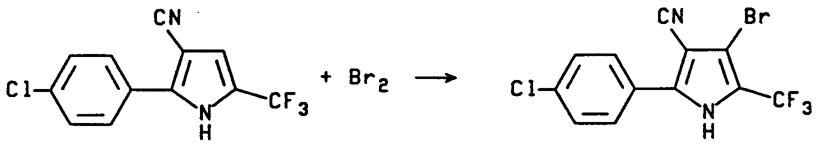

A solution of 2-(p-chlorophenyl)-5-(trifluoromethyl)pyrrole-3-carbonitrile (1 equivalent) in carbon tetrachloride is treated with bromine (1.8 equivalents), stirred at ambient temperatures until the reaction is complete and concentrated in vacuo to give the title product in quantitative yield, identified by IR, NMR and mass spectral analyses.

### EXAMPLE 7

### Preparation of 4-bromo-2-(p-chlorophenyl)-1-(ethoxymethyl)-5-(trifluoromethyl)pyrrole-3-carbonitrile

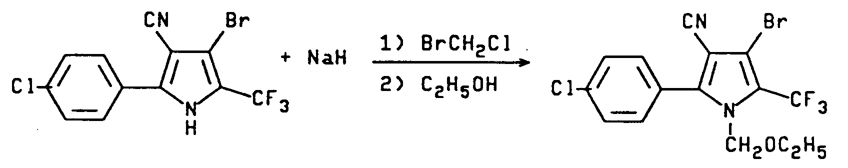

A stirred mixture of sodium hydride (26.5 g, 1.105 mol) in tetrahydrofuran, under nitrogen, is treated slowly with a solution of 4-bromo-2-(p-chlorophenyl)-5-(trifloromethyl)pyrrole-3-carbonitrile (1.00 g, 0.273 mol) in tetrahdrofuran at ≦60°C. When the addition is complete, the reaction mixture is treated with bromochloromethane (93.0 g, 0.718 mol), heated to 63°-65°C, treated dropwise with absolute ethanol (39.5 g, 0.859 mol) over a 5.5 hour period, stirred at 63°-65°C for another 3 hours and concentrated in vacuo. The concentrate is dispersed in a mixture of water and methylene chloride. The aqueous phase is separated and washed with methylene chloride. The organic phases are combined and concentrated. The concentrate is dispersed in hexane, cooled to 5°-10°C and filtered. The filtercake is washed as a white solid, 111.6 g, (91.3% yield) and 91.2% purity by HPLC analysis.

## Claims

1. A method for the preparation of a 2-aryl-5-trifluoromethyl pyrrole compound of formula I 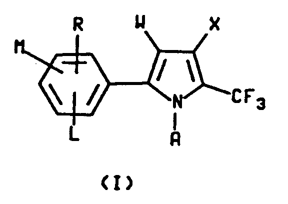 wherein
A is hydrogen or C₁-C₆ alkyl optionally substituted with phenyl;
W is CN, NO₂, CO₂R₁ or SO₂R₁;
X is hydrogen or CO₂R₂;
L is hydrogen, F, Cl, Br or I;
M and R are each independently hydrogen, C₁-C₄ alkyl, C₁-C₄ alkoxy, C₁-C₄ alkylthio, C₁-C₄ alkylsulfinyl, C₁-C₄ alkylsulfonyl, CN, F, Cl, Br, I, NO₂, CF₃, R₃CF₂Z, R₄CO or NR₅R₆ and when M and R are on adjacent postions they may be taken together with the carbon atoms to which they are attached to form a ring in which MR represents the structure
-OCH₂O-, -OCF₂O- or -CH=CH-CH=CH-;
R₁ is C₁-C₆ alkyl, C₃-C₆ cycloalkyl or phenyl;
R₂ is C₁-C₄ alkyl;
R₃ is hydrogen, F, CHF₂, CHFCl or CF₃;
R₄ is C₁-C₄ alkyl, C₁-C₄ alkoxy or NR₅R₆;
R₅ is hydrogen or C₁-C₄ alkyl;
R₆ is hydrogen, C₁-C₄ alkyl or R₇CO;
R₇ is hydrogen or C₁-C₄ alkyl;
Z is S(O)ₙ or O; and
n is an integer of 0, 1 or 2
which comprises reacting a haloenamine of formula II 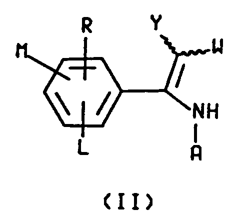 wherein Y is Cl, Br or I, and A, W, L, M and R are as described above with about one molar equivalent of trifluoroacetone or C₁-C₄ trifluoroacetoacetate in the presence of an acid and optionally in the presence of a solvent.

2. The method according to claim 1 wherein the reaction takes place at an elevated temperature.

3. The method according to claim 1 wherein the acid is acetic acid.

4. The method according to claim 1 wherein the formula II haloenamine is reacted with about one molar equivalent of trifluroacetone.

5. The method according to claim 1 wherein the formula II haloenamine is reacted with about one molar equivalent of C₁-C₄ alkyl trifluoroacetoacetate.

6. The method according to claim 2 wherein the elevated temperature is about 60°C to 250°C.

7. The method according to claim 3 wherein the reaction takes place at an elevated temperature of about 70°C to 180°C.

8. The method according to claim 4 wherein the reaction takes place at an elevated temperature of about 70°-180°C.

9. The method according to claim 1 wherein W is CN.

10. The method according to claim 9 wherein A is hydrogen or C₁-C₆ alkyl.

## Patentansprüche

1. Verfahren zur Herstellung einer 2-Aryl-5-trifluormethylpyrrolverbindung der Formel I 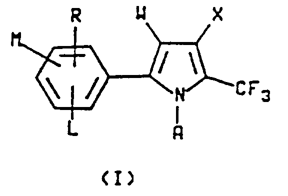 wobei
A für Wasserstoff oder gegebenenfalls mit Phenyl substituiertes C₁-C₆-Alkyl steht;
W für CN, NO₂, CO₂R₁ oder SO₂R₁ steht;
X für Wasserstoff oder CO₂R₂ steht;
L für Wasserstoff, F, Cl, Br oder I steht;
M und R jeweils unabhängig voneinander für Wasserstoff, C₁-C₄-Alkyl, C₁-C₄-Alkoxy, C₁-C₄-Alkylthio, C₁-C₄-Alkylsulfinyl, C₁-C₄ Alkylsulfonyl, CN, F, Cl, Br, I, NO₂, CF₃, R₃CF₂Z, R₄CO oder NR₅R₆ stehen, und falls M und R in benachbarten Positionen vorliegen, können sie zusammen mit den Kohlenstoffatomen, an welche sie gebunden sind, einen Ring bilden, wobei MR für die Struktur
-OCH₂O-, -OCF₂O- oder -CH=CH-CH=CH- steht;
R₁ für C₁-C₆-Alkyl, C₃-C₆-Cycloalkyl oder Phenyl steht;
R₂ für C₁-C₄-Alkyl steht;
R₃ für Wasserstoff F, CHF₂, CHFCl oder CF₃ steht;
R₄ für C₁-C₄-Alkyl, C₁-C₄-Alkoxy oder NR₅R₆ steht;
R₅ für Wasserstoff oder C₁-C₄-Alkyl steht;
R₆ für Wasserstoff C₁-C₄-Alkyl oder R₇CO steht;
R₇ für Wasserstoff oder C₁-C₄-Alkyl steht;
Z für S(O)ₙ oder O steht; und
n eine ganze Zahl aus 0, 1 oder 2 bedeutet;
wobei das Verfahren umfaßt eine Umsetzung eines Halogen-Enamins der Formel II 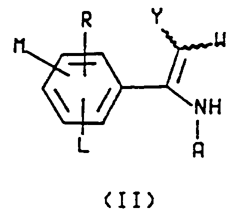 wobei Y für Cl, Br oder I steht, und A, W, L, M und R wie oben beschrieben sind, mit etwa einem molaren Äquivalent Trifluoraceton oder C₁-C₄-Alkyltrifluoracetacetat in Gegenwart einer Säure und gegebenenfalls in Gegenwart eines Lösungsmittels.

2. Verfahren gemäß Anspruch 1, wobei die Reaktion bei einer erhöhten Temperatur stattfindet.

3. Verfahren gemäß Anspruch 1, wobei die Säure Essigsäure ist.

4. Verfahren gemäß Anspruch 1, wobei das Halogen-Enamin der Formel II mit etwa einem molaren Äquivalent Trifluoraceton umgesetzt wird.

5. Verfahren gemäß Anspruch 1, wobei das Halogen-Enamin der Formel II mit etwa einem molaren Äquivalent C₁-C₄-Alkyltrifluoracetacetat umgesetzt wird.

6. Verfahren gemäß Anspruch 2, wobei die erhöhte Temperatur etwa 60° bis 250°C beträgt.

7. Verfahren gemäß Anspruch 3, wobei die Reaktion bei einer erhöhten Temperatur von etwa 70 bis 180°C stattfindet.

8. Verfahren gemäß Anspruch 4, wobei die Reaktion bei einer erhöhten Temperatur von etwa 70 bis 180°C stattfindet.

9. Verfahren gemäß Anspruch 1, wobei W für CN steht.

10. Verfahren gemäß Anspruch 9, wobei A für Wasserstoff oder C₁-C₆-Alkyl steht.

## Revendications

1. Procédé de préparation d'un composé de type 2-aryl-5-trifluorométhyl pyrrole de formule I : 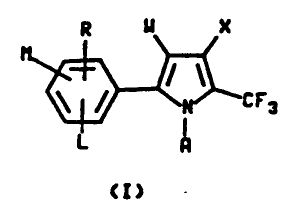 dans laquelle :
A représente un atome d'hydrogène ou un groupe alkyle en C₁-C₆ éventuellement substitué par un groupe phényle ;
W représente CN, NO₂, CO₂R₁ ou SO₂R₁ ;
X représente un atome d'hydrogène ou CO₂R₂ ;
L représente un atome d'hydrogène, F, Cl, Br ou I ;
M et R représentent indépendamment chacun un atome d'hydrogène, un groupe alkyle en C₁-C₄, alkoxy en C₁-C₄, alkylthio en C₁-C₄, alkylsulfinyle en C₁-C₄, alkylsulfonyle en C₁-C₄, CN, F, Cl, Br, I, NO₂, CF₃, R₃CF₂Z, R₄CO ou NR₅R₆, et lorsque M et R sont en positions adjacentes, ils peuvent former ensemble avec les atomes de carbone auxquels ils sont liés, un cycle dans lequel MR représente la structure :
-OCH₂O-, -OCF₂O- ou -CH=CH-CH=CH- ;
R₁ représente un groupe alkyle en C₁-C₆, cycloalkyle en C₃-C₆ ou phényle ;
R₂ représente un groupe alkyle en C₁-C₄ ;
R₃ représente un atome d'hydrogène, F, CHF₂, CHFCl ou CF₃ ;
R₄ représente un groupe alkyle en C₁-C₄ , alkoxy en C₁-C₄ ou NR₅R₆ ;
R₅ représente un atome d'hydrogène ou un groupe alkyle en C₁-C₄ ;
R₆ représente un atome d'hydrogène, un groupe alkyle en C₁-C₄ ou R₇CO ;
R₇ représente un atome d'hydrogène ou un groupe alkyle en C₁-C₄ ;
Z représente S(O)n ou O ; et
n représente un entier égal à 0, 1 ou 2 ;
selon lequel on fait réagir une halogénoénamine de formule II : 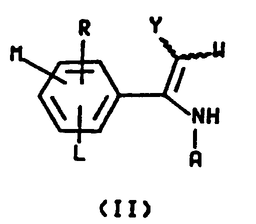 dans laquelle Y représente Cl, Br ou I, et A, W, L, M et R sont sels que définis ci-dessus, avec environ un équivalent molaire de trifluoroacétone ou d'un trifluoroacétoacétate d'alkyle en C₁-C₄, en présence d'un acide, et éventuellement en présence d'un solvant.

2. Procédé selon la revendication 1, dans lequel la réaction est effectuée à température élevée.

3. Procédé selon la revendication 1, dans lequel l'acide est l'acide acétique.

4. Procédé selon la revendication 1, dans lequel on fait réagir l'halogénoénamine de formule II avec environ 1 équivalent molaire de trifluoroacétone.

5. Procédé selon la revendication 1, dans lequel on fait réagir l'halogénoénamine de formule II avec environ 1 équivalent molaire de trifluoroacétoacétate d'alkyle en C₁-C₄.

6. Procédé selon la revendication 2, dans lequel la température élevée est d'environ 60 °C à 250 °C.

7. Procédé selon la revendication 3, dans lequel la réaction est effectuée à une température élevée d'environ 70 °C à 180 °C.

8. Procédé selon la revendication 4, dans lequel la réaction est effectuée à une température élevée d'environ 70° à 180 °C.

9. Procédé selon la revendication 1, dans lequel W représente CN.

10. Procédé selon la revendication 9, dans lequel A représente un atome d'hydrogène ou un groupe alkyle en C₁-C₆.
